# EUROPEAN PATENT APPLICATION

(11) **EP 2 463 273 A1**
(43) Date of publication of application: **13.06.2012**
(21) Application number: 11460062.0
(22) Date of filing: 09.12.2011
(51) Int. Cl.: C07C 263/10, C07C 263/20, C07C 265/14

(54) **Method for separating toluene diisocyanate from the post-reaction mixture in the toluene diamine phosgenation process in the gas phase**

(30) Priority: 10.12.2010 PL 39321410
(71) Applicant: Zaklady Chemiczne ZACHEM S.A., 85-825 Bydgoszcz (PL); POLITECHNIKA WARSZAWSKA, 00-661 Warszawa (PL); Instytut Chemii Przemyslowej im. Prof. Ignacego Moscickiego, 01-793 Warszawa (PL)
(72) Inventor: Baldyga, Jerzy, 05-540 Zalesie Górne (PL); Henczka, Marek, 02-792 Warszawa (PL); Szarlik, Stefan, 05-082 Blizne Laszczynskiego (PL); Ilmurzynska, Janina, 02-777 Warszawa (PL); Dyczewski, Michal, 05-420 Józefów (PL); Slawatycki, Arkadiusz, 85-861 Bydgoszcz (PL); Chrupala, Wojciech, 85-704 Bydgoszcz (PL); Lachmajer, Jerzy, 85-149 Bydgoszcz (PL); Ruczynski, Lech, 85-565 Bydgoszcz (PL); Wójcik, Lucjan, 86-801 Niemcz (PL); Stuczynski, Jacek, 85-801 Bydgoszcz (PL)
(74) Representative: Twardowska, Aleksandra

(57) **Abstract**

The present invention relates to the method for separating toluene diisocyanate from the post-reaction mixture in the toluene diamine phosgenation process in the gas phase. More specifically the solution concerns obtaining a higher TDI concentration in the liquid reaction product. The method is based on the fact that cooling and condensation of the reaction product - toluene diisocyanate - (TDI) is carried out by the application of spraying in the cooling and condensation zone, directly after the reactor, wherein this operation is carried out at a temperature elevated enough to obtain a low concentration of the solvent, i.e. within 5-30% by weight, in relation to TDI obtained, and thus a high concentration of (within 70-95% by weight) TDI obtained.

## Description

The invention relates to the method for separating toluene diisocyanate from the post-reaction mixture in the toluene diamine phosgenation process in the gas phase. More specifically, the solution relates to obtaining a higher concentration of TDI in the liquid reaction product than in the prior art. The method is based on the fact that cooling and condensation of the reaction product - toluene diisocyanate (TDI) - is carried out by the application of spraying in the cooling and condensation zone, directly after the reactor, wherein this operation is carried out at a temperature elevated enough to obtain a low concentration of the solvent in the condensed liquid, i.e. within 5-30% by weight, in relation to TDI obtained, and thus high concentration of (within 70-95% by weight) TDI obtained.

Carrying out the toluene diamine phosgenation process in the gas phase allows for reducing the amount of solvent used, mostly dichlorobenzene (DCB), by about 80%, thus decreasing the energy consumption by about 40% in comparison to the traditional method of TDA phosgenation in the liquid phase (Urethane chemicals process enters a new phase 19.08.2008, www.processengineering.com.uk).

In the most well-known process by the Bayer company, described in such patent publications as US/2008 0146834 and US 2005/0272910 A1, gaseous TDA and gaseous phosgene, in the presence of the inert gas, contact each other in the reactor chamber, in adiabatic conditions, with a subsequent cooling of the gaseous product stream that is leaving the reactor, to a temperature above the decomposition temperature of carbamoyl chloride for isocyanate condensation. Uncondensed isocyanate is then separated from the stream of waste gas by means of absorption in a solution containing mainly a solvent and TDI. The gaseous mixture, after cooling and condensation of isocyanate, is purified from the remaining isocyanate in the scrubber, in which an inert liquid solvent is used, such as o-DCB (ortho-dichlorobenzene). The phosgene is released from the stream of liquid that leaves the scrubber, e.g. by cooling and absorption in a suitable solvent or adsorption on activated carbon. The solvent that leaves the scrubber is used as a coolant for the gas stream leaving the reaction chamber. The schematic diagram of the Bayer process is shown in Pos. 1.

Despite the existence of solutions known in the art, there is still a need for solutions that allow one to achieve a higher concentration of TDI in the liquid reaction product compared to concentrations obtained in the prior art.

The aim of the present invention is to provide such a method for separating toluene diisocyanate from the post-reaction mixture in the toluene diamine phosgenation process in the gas phase, which allows one to achieve concentrations of TDI in the liquid reaction product that are higher than those provided in the prior art, wherein the cooling and condensation of the reaction product, i.e. TDI, is conducted by the application of spraying in the cooling and condensation zone, directly after the reactor, wherein this operation is carried out at a temperature elevated enough to obtain a low concentration of the solvent in the condensed liquid, i.e. within 5-30% by weight, in relation to TDI obtained, and thus a high concentration of (within 70-95% by weight) TDI obtained.

The proposed method for conducting the process of TDI separation from the reaction products, after the toluene diamine phosgenation in the gas phase, will reduce the amount of solvent used, simultaneously reducing energy consumption compared to methods used in classical TDA phosgenation in the liquid phase and solutions previously used for TDI separation after the reaction in the gas phase.

The essential feature of the invention is that toluene diisocyanate formed as a reaction product is condensed from gaseous reaction products in such a manner that spraying the stream of post-reaction gases with a liquid solvent, most often dichlorobenzene, provides a cooling of post-reaction gases to a temperature at which the condensed liquid contained only 5-30% by weight of solvent, preferably up to 10% by weight, while maintaining a condensed product at this temperature should be as short as possible in order not to cause TDI decomposition.

The invention relates to the method for separating toluene diisocyanate from the post-reaction mixture in the phosgenation process of toluene diamine, in the gas phase, characterised in that the stream of post-reaction gases is being sprayed by a liquid solvent in the cooling zone to temperature at which the condensed liquid contains 5-30% by weight of solvent, and wherein toluene diisocyanate TDI, separated from gaseous reaction products, is obtained.

Preferably, the cooling and condensation of the reaction product - toluene diisocyanate - is carried out by the application of spraying in the cooling and condensation zone, directly after the reactor, wherein this operation is carried out at a temperature elevated enough to obtain a concentration of the solvent in the condensed liquid, within 5-30% by weight, in relation to TDI obtained.

Preferably, the liquid solvent for spraying the stream of post-reaction gases is dichlorobenzene.

Preferably, the condensed liquid contains up to 10% by weight of the solvent. Preferably, the gases leaving the TDI cooling and condensation zone, directly after the reactor, are cooled by means of jacket cooling in order to condense the residual solvent, and subsequently, these gases are expanded and directed towards the recovery node for excess phosgene.

Preferably, a condensed reaction product containing up to 30% by weight of solvent is overheated by 30-40°C in a heater and expanded near atmospheric pressure, thereby obtaining the disengagement of substances dissolved in the liquid product, especially phosgene, and furthermore hydrogen chloride and nitrogen, and partly the solvent, wherein the stream of obtained gases is subjected to indirect condensation of the solvent in the surface condenser, and this solvent collects in the separator, to which also flows the solvent condensed earlier in the surface condenser, from post-reaction gases, after being separated from the post reaction gases in the separator. The whole condensed solvent is recycled as a coolant for the gaseous post-reaction mixture.

Preferably, the phosgenation reaction is carried out in an adiabatic manner, under a pressure of 400 kPa (a), then the gaseous post-reaction mixture is cooled to a temperature of 200-215°C, wherein maintaining the condensed product at that temperature should be short in order not to cause TDI decomposition.

Preferably, the gaseous post-reaction mixture is cooled at that time to a temperature of 210°C.

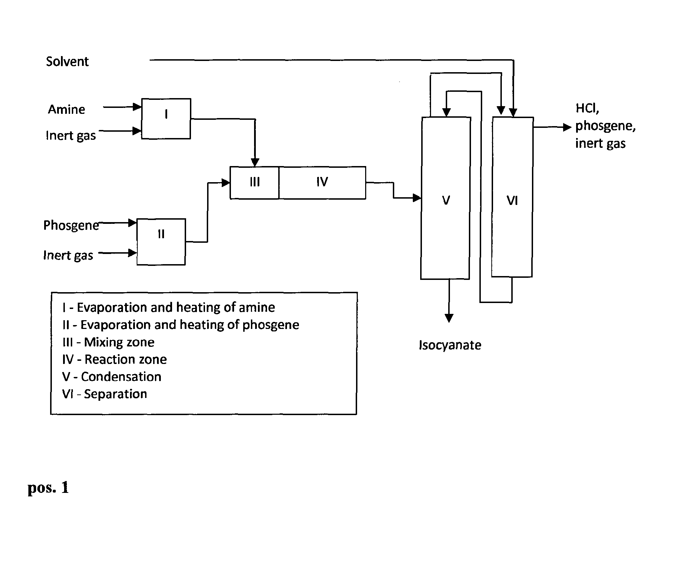

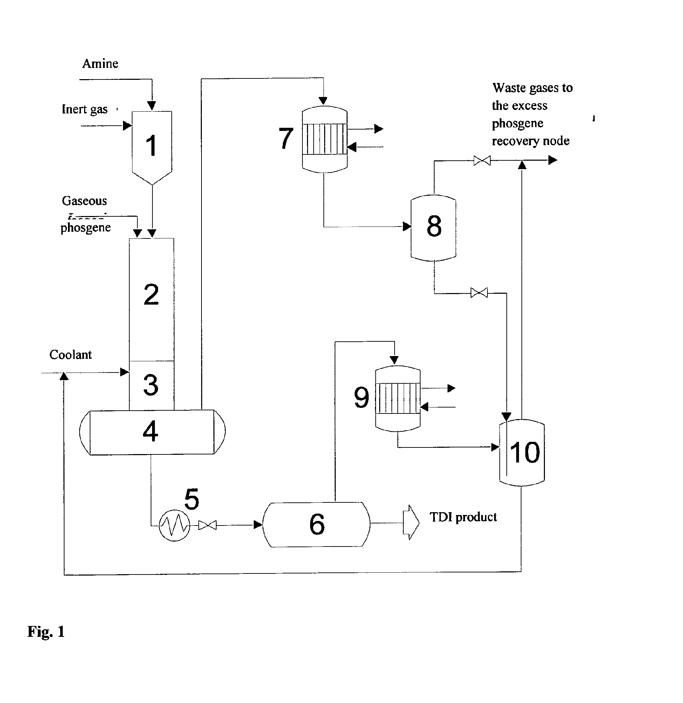

For better understanding of the invention:
**Pos. 1** presents a schematic diagram of the Bayer process, while **figure 1** presents the scheme of the process, where
   1. TDA evaporator
   2. Reactor
   3. Cooling zone
   4. Receiver of the liquid product
   5. Heater
   6. Receiver
   7, 9. Condensers
   8, 10. Separators.

The following examples illustrate implementation of the process of obtaining TDI by TDA phosgenation in the gas phase, without limiting the scope of the invention.

### Example 1

In the TDA evaporator 1, 5.0 kg/h TDA was evaporated in a continuous manner to a stream of 2.3 kg/h of flowing nitrogen. Phosgenation of the obtained gaseous TDA stream, prepared by evaporation to a nitrogen flow-through stream of liquid amine, was carried out by the contact of the previously mentioned amine vapours and gaseous phosgene in the flow-through reactor 2. The reaction was carried out in adiabatic conditions at a pressure of 400 kPa (a). As a result of the reaction, the temperature rose from 300°C to 382°C. There was a total consumption of the TDA observed. Post-reaction gases, containing TDI, excess phosgene, hydrogen chloride and inert nitrogen, were subjected to cooling, in the cooling zone 3, with a stream of approximately 13 kg/h of the coolant at a temperature of 30°C, which the stream was comprised of a mixture of o-DCB and TDI with a relevant composition 68/32. As a result of cooling, TDI was condensed to the receiver of the liquid product 4, which gave a liquid containing 79.82% of TDI by weight and 19.79% oDCB by weight. The condensed liquid also contained dissolved: phosgene, nitrogen and hydrogen chloride, with a total amount of approximately 0.4% by weight.

### Example 2

In the TDA evaporator 1, 5.0 kg/h TDA was evaporated in a continuous manner to a stream of 2.3 kg/h of flowing nitrogen. Phosgenation of the obtained gaseous TDA stream, prepared by evaporation to the nitrogen flow-through stream of liquid amine, was carried out by the contact of the previously mentioned amine vapours and gaseous phosgene in the flow-through reactor 2. The reaction was carried out in adiabatic conditions at a pressure of 400 kPa (a). As a result of the reaction, the temperature rose from 300°C to 382°C. There was a total consumption of the TDA observed. Post-reaction gases, containing TDI, excess phosgene, hydrogen chloride and inert nitrogen, were cooled in the cooling zone 3 analogously, as in Example 1, by being sprayed by a coolant at a temperature of 30°C using a smaller amount of coolant, approximately 11 kg/h. Cooling was done only to a temperature of 215°C. The resulting liquid reaction product contained 91.00% TDI by weight and 8.66% oDCB by weight, while the residue, up to 100%, was made up by HCl, phosgene and nitrogen dissolved in the liquid.

## Claims

1. A method for separating toluene diisocyanate from the post-reaction mixture in the toluene diamine phosgenation process in the gas phase, **characterised in that** the stream of post-reaction gases is sprayed with a liquid solvent in the cooling zone (3) post-reaction gases are cooled to a temperature at which the condensed liquid contains 5-30% by weight of solvent, and toluene diisocyanate TDI, condensed from gaseous reaction products, is obtained.

2. A method according to claim 1, **characterised in that** the cooling and condensation of the reaction product - toluene diisocyanate - is performed by the application of spraying in the cooling and condensation zone, directly after the reactor (2), wherein this operation is carried out at a temperature elevated enough to obtain a concentration of the solvent within 5-30% by weight in relation to TDI obtained.

3. A method according to claim 1, **characterised in that** liquid solvent for spraying with a stream of post-reaction gases is dichlorobenzene.

4. A method according to claim 1, **characterised in that** the condensed liquid contains up to 10% by weight of the solvent.

5. A method according to claim 1, **characterised in that** the gases leaving the TDI cooling and condensation zone, directly after the reactor, are cooled by means of jacket cooling, and subsequently, these gases are expanded and directed towards the recovery node for excess phosgene.

6. A method according to claim 1, **characterised in that** the condensed reaction product containing up to 30% by weight of solvent is overheated by 30-40°C and expanded at near atmospheric pressure, obtaining disengagement of substances dissolved in the liquid product, especially phosgene, hydrogen chloride and nitrogen, and partly the solvent, wherein the stream of obtained gases is subjected to indirect condensation of solvent, which, being combined with the solvent condensed earlier from post-reaction gases, is recycled as a coolant for the post reaction gaseous mixture after the reactor.

7. A method according to claim 1, **characterised in that** when the phosgenation reaction is carried out in an adiabatic manner under a pressure of 400 kPa (a), the gaseous post-reaction mixture is then cooled to a temperature of 200-215°C.

8. A method according to claim 1 and 7, **characterised in that** the gaseous post-reaction mixture is cooled to a temperature of 210°C.
